# EUROPEAN PATENT APPLICATION

(11) **EP 4 552 582 A1**
(43) Date of publication of application: **14.05.2025**
(21) Application number: 24211585.5
(22) Date of filing: 07.11.2024
(51) Int. Cl.: A61B 8/08, A61B 8/00, G06N 3/08, G16H 30/40, G16H 50/20

(54) **ULTRASOUND DIAGNOSTIC APPARATUS**

(30) Priority: 08.11.2023 JP 2023190801
(71) Applicant: FUJIFILM Corporation, Tokyo 106-8620 (JP)
(72) Inventor: DANNO, Natsumi, Tokyo, 106-8620 (JP); TSUJITA, Takehiro, Tokyo, 106-8620 (JP); IKEDA, Teiichiro, Tokyo, 106-8620 (JP)
(74) Representative: Dehns Germany Partnerschaft mbB

(57) **Abstract**

Provided is an ultrasound diagnostic apparatus (10) capable of adaptively outputting support information according to a cross-section classification of a formed ultrasound tomographic image and a targeted cross-section. In a support information DB, comments as different pieces of support information are associated with a plurality of different match rates from each other. A targeted cross-section specifying unit (36) specifies a cross-section type of an ultrasound tomographic image that needs to be formed by an examiner, that is, a targeted cross-section. A match rate calculation unit (38) calculates a match rate between a target image, which is an ultrasound tomographic image formed by transmitting ultrasound waves to a subject, and the targeted cross-section specified by the targeted cross-section specifying unit (36). An output unit (20) outputs, based on the support information DB, support information with a content tailored to the match rate between a cross-section classification of the target image and the targeted cross-section, which is calculated by the match rate calculation unit (38).

## Description

### BACKGROUND OF THE INVENTION

### 1. Field of the Invention

The present specification discloses improvement of an ultrasound diagnostic apparatus.

### 2. Description of the Related Art

An ultrasound diagnostic apparatus is an apparatus that forms an ultrasound image or performs various examinations based on reflected waves from a subject, which are obtained by irradiating the subject with ultrasound waves. Conventionally, an ultrasound diagnostic apparatus has a function of supporting an examiner (user) such as a doctor or a technician.

For example, JP2022-127808A discloses an ultrasound diagnostic apparatus that stores an examination protocol in which a series of examination processes and examination execution conditions for each examination process are set, in which the execution conditions are automatically set for each examination process in a case where an examiner selects a certain examination protocol, and the examination process automatically transitions to the next examination process in a case where an examination necessary for the certain examination process has ended.

### SUMMARY OF THE INVENTION

Meanwhile, in an examination (including measurement) using an ultrasound diagnostic apparatus, it may be necessary to form an ultrasound tomographic image of an appropriate cross-section according to the purpose of the examination. In the present specification, an appropriate cross-section tailored to an examination is referred to as a targeted cross-section. The targeted cross-section is, for example, defined in information such as the examination protocol disclosed in JP2022-127808A or input to the ultrasound diagnostic apparatus by the examiner. In order to correctly form the ultrasound tomographic image of the targeted cross-section, it is necessary to correctly apply an ultrasound probe to a correct position of the subject in a correct orientation and to correctly perform settings of the ultrasound diagnostic apparatus. In the present specification, the method of applying the ultrasound probe to the subject, the settings of the ultrasound diagnostic apparatus, and the like are collectively referred to as an operation of the ultrasound diagnostic apparatus (by the examiner).

Here, in order to support the formation of the ultrasound tomographic image of the targeted cross-section, it is considered to provide the examiner with support information related to the operation of the ultrasound diagnostic apparatus. As a simple method, it is conceivable to provide the examiner with uniform support information tailored to the targeted cross-section. However, it may not be appropriate to provide uniform support information to the examiner. For example, a plurality of examiners attempting to form the ultrasound images of the same targeted cross-section may form ultrasound images of different cross-section classifications depending on their level of proficiency in operation. For example, while an examiner who has a relatively high level of proficiency in operation but still requires support may be able to form an ultrasound image of a cross-section classification close to the targeted cross-section, an examiner who has a considerably low level of proficiency in operation may form an ultrasound image of a cross-section that is quite far from the targeted cross-section. In such a case, it is not appropriate to uniformly provide the same support information to both parties.

An object of the ultrasound diagnostic apparatus disclosed in the present specification is to provide an ultrasound diagnostic apparatus capable of adaptively outputting support information according to a cross-section classification of a formed ultrasound tomographic image and a targeted cross-section.

An ultrasound diagnostic apparatus disclosed in the present specification comprises: a targeted cross-section specifying unit that specifies a targeted cross-section which is a cross-section classification of an ultrasound tomographic image that needs to be formed; a match rate calculation unit that executes cross-section recognition processing on a target image, which is an ultrasound tomographic image formed in response to an instruction from an examiner, to calculate a match rate between a cross-section classification of the target image and the targeted cross-section; and an output unit that outputs, based on a support information database in which support information related to an operation of the ultrasound diagnostic apparatus and used to support formation of a targeted cross-section, which is a cross-section classification of an ultrasound tomographic image that needs to be formed by the examiner, is associated with each match rate, support information with a content tailored to the match rate calculated by the match rate calculation unit.

In the support information database, the support information may be associated with each combination of the match rate and a display aspect of the target image, and the output unit may output support information with a content tailored to the match rate calculated by the match rate calculation unit and to the display aspect of the target image.

In the support information database, the support information may be associated with a combination of the match rate and a measurement content using the target image, and the output unit may output support information with a content tailored to the match rate calculated by the match rate calculation unit and to the measurement content.

In the support information database, the support information may be associated with a combination of the match rate and an operation mode of the ultrasound diagnostic apparatus, and the output unit may output support information with a content tailored to the match rate calculated by the match rate calculation unit and to the operation mode of the ultrasound diagnostic apparatus.

In the support information database, the support information may be associated with a combination of the match rate and an attribute of a subject, and the output unit may output support information with a content tailored to the match rate calculated by the match rate calculation unit and to the attribute of the subject.

In the support information database, the support information may be associated with a combination of the match rate, a first cross-section classification, and a second cross-section classification other than the first cross-section classification, and the output unit may output support information with a content tailored to the match rate calculated by the match rate calculation unit, to the cross-section classification of the target image as the first cross-section classification, and to the targeted cross-section as the second cross-section classification.

A support information editing unit that edits the support information in response to an instruction from a user of the ultrasound diagnostic apparatus may be further provided.

The match rate calculation unit may calculate the match rate for the same targeted cross-section again after the support information has been output, and assign, in a case where the match rate after the support information has been output is higher than the match rate before the support information is output, a priority flag to the support information in the support information database, and the output unit may preferentially output the support information with the priority flag assigned, in a case where a plurality of pieces of the support information are associated with the match rate in the support information database, over support information with no priority flag assigned.

During an examination along a plurality of examination processes, each forming an ultrasound tomographic image of a different targeted cross-section, the match rate calculation unit may calculate the match rate for each examination process and store the match rate in a memory, and the output unit may output the calculated match rate for a completed examination process during the examination along the plurality of examination processes.

The output unit may output, after a plurality of examination processes, each forming an ultrasound tomographic image of a different targeted cross-section, have ended, an examination report that includes, for each of the plurality of examination processes, item values for a plurality of items including the match rate and the support information output in a case of attempting to form an ultrasound tomographic image of a targeted cross-section related to the examination process, the examination report associating information on the item tailored to the match rate corresponding to each targeted cross-section with the targeted cross-section.

According to the ultrasound diagnostic apparatus disclosed in the present specification, it is possible to provide an ultrasound diagnostic apparatus capable of adaptively outputting support information according to a cross-section classification of a formed ultrasound tomographic image and a targeted cross-section.

### BRIEF DESCRIPTION OF THE DRAWINGS

Fig. 1 is a schematic diagram of a configuration of an ultrasound diagnostic apparatus according to the present embodiment.
Fig. 2 is a conceptual diagram showing an example of a content of a support information DB.
Fig. 3 is a diagram showing a first example of a reference image as support information.
Fig. 4 is a diagram showing a second example of the reference image as the support information.
Fig. 5 is a diagram showing an example of a support information display screen.
Fig. 6 is a diagram showing an example of an examination report.

### DESCRIPTION OF THE PREFERRED EMBODIMENTS

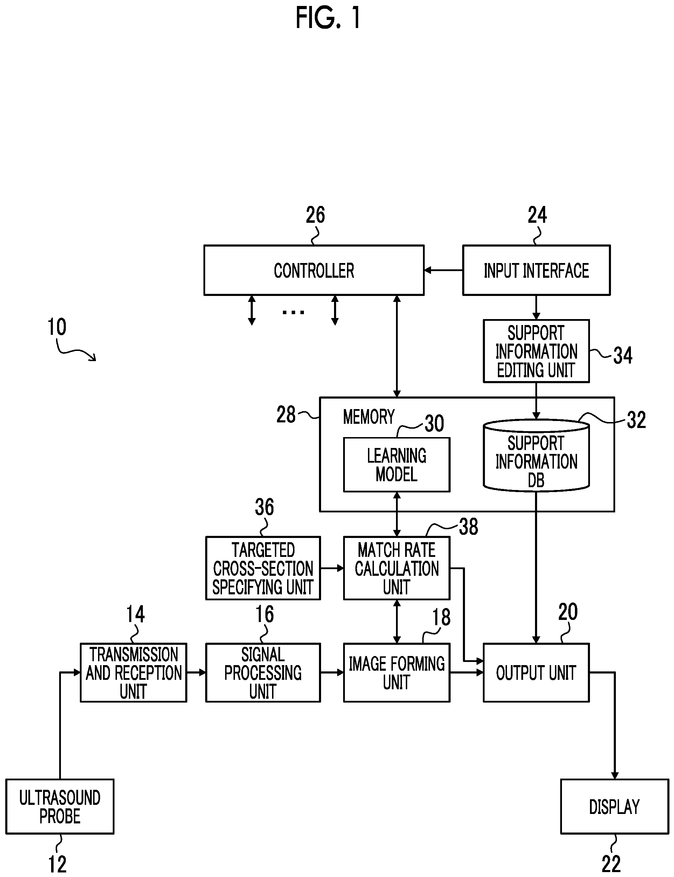

Fig. 1 is a schematic diagram of a configuration of an ultrasound diagnostic apparatus 10 according to the present embodiment. The ultrasound diagnostic apparatus 10 is a medical apparatus installed in a medical institution such as a hospital and used during ultrasound examinations.

The ultrasound diagnostic apparatus 10 is an apparatus that scans a subject with an ultrasound beam and that generates an ultrasound image based on a reception signal obtained by the scanning. The ultrasound diagnostic apparatus 10 has, for example, a plurality of operation modes such as a B-mode, an M-mode, a pulse wave (PW) Doppler mode, a continuous wave (CW) Doppler mode, or a color Doppler mode. For example, in the B-mode, the ultrasound diagnostic apparatus 10 forms an ultrasound tomographic image (B-mode image) in which the amplitude intensity of a reflected wave from a scanning plane is converted into brightness, based on the reception signal. In addition, for example, in the PW Doppler mode, the CW Doppler mode, or the color Doppler mode, the ultrasound diagnostic apparatus 10 forms a Doppler image that shows the motion velocity of a site of the subject or the velocity of the blood flow, based on the difference in frequency between transmission ultrasound waves and reception ultrasound waves. The Doppler image shows the motion velocity of the site of the subject or the velocity of the blood flow in colors or the like and is superimposed and displayed on the ultrasound tomographic image. An operation mode of the ultrasound diagnostic apparatus 10 can be set in response to an instruction from the examiner.

Although details will be described below, the ultrasound diagnostic apparatus 10 can provide the examiner with support information related to an operation of the ultrasound diagnostic apparatus 10 and used to support formation of a targeted cross-section which is a cross-section classification of an ultrasound tomographic image that needs to be formed by the examiner. For example, in a case where a beginner or the like who is not familiar with the operation of the ultrasound diagnostic apparatus 10 operates the ultrasound diagnostic apparatus 10, the ultrasound diagnostic apparatus 10 can exhibit a function of providing the support information.

An ultrasound probe 12 is a device that performs transmission and reception of ultrasound waves with respect to the subject. The ultrasound probe 12 includes a transducer element array consisting of a plurality of transducer elements that perform transmission and reception of ultrasound waves with respect to the subject.

A transmission and reception unit 14 transmits a transmission signal to the ultrasound probe 12 (in detail, each transducer element of the transducer element array) under the control of a controller 26 (which will be described below). Consequently, the ultrasound waves are transmitted from each transducer element toward the subject. In addition, the transmission and reception unit 14 receives the reception signal from each transducer element that has received the reflected wave from the subject. The transmission and reception unit 14 includes an adder and a plurality of delay devices corresponding to the respective transducer elements and performs phase alignment addition processing of aligning and adding phases of the reception signals from the respective transducer elements by using the adder and the plurality of delay devices. As a result, reception beam signals are formed in which information indicating the signal intensity of the reflected wave from the subject is arranged in a depth direction of the subject.

A signal processing unit 16 executes various types of signal processing including filter processing of applying a bandpass filter, detection processing, and the like on the reception beam signals from the transmission and reception unit 14. In addition, in a case where the operation mode of the ultrasound diagnostic apparatus 10 is the Doppler mode, the signal processing unit 16 generates Doppler data indicating the site of the subject or the velocity of the blood flow based on the reception beam signals from the transmission and reception unit 14. Specifically, the signal processing unit 16 generates the Doppler data by performing processing such as quadrature detection of multiplying the reception beam signal by a reference frequency (transmission frequency) to extract Doppler shifts through a low-pass filter, sample gate processing (in a case of the PW Doppler mode) of extracting only the signals from the position of the sample volume, A/D conversion of the signals, and frequency analysis using the fast Fourier transform method (FFT method).

An image forming unit 18 forms the ultrasound tomographic image (B-mode image) based on the reception beam signal subjected to the signal processing in the signal processing unit 16. First, the image forming unit 18 converts the reception beam signal into data on a coordinate space of the ultrasound image and forms the ultrasound tomographic image based on a coordinate conversion signal. Additionally, in a case where the operation mode of the ultrasound diagnostic apparatus 10 is the Doppler mode, the image forming unit 18 forms a Doppler waveform or a color Doppler image based on a Doppler signal from the signal processing unit 16.

An output unit 20 performs control to display the ultrasound image, such as the ultrasound tomographic image or the color Doppler image formed by the image forming unit 18, on a display 22. The display 22 is a display device configured with, for example, a liquid crystal display, an organic electro luminescence (EL), or the like.

The output unit 20 can display the ultrasound image (the ultrasound tomographic image, the Doppler image, or the like) on the display 22 in either real-time or freeze display aspect. The display aspect of the ultrasound image can be switched in response to an instruction from the examiner. In a case of the real-time display aspect, the output unit 20 displays, on the display 22 in real-time, the ultrasound images that are successively formed by the processing of the signal processing unit 16 and the image forming unit 18 in response to the reception signals that are successively received by the ultrasound probe 12. Meanwhile, in a case of the freeze display aspect, the output unit 20 displays, on the display 22, the ultrasound image formed at the timing as instructed by the examiner, as a still image.

In addition, although details will be described below, the output unit 20 displays the support information on the display 22. The output unit 20 may output the support information as a voice from a speaker (not shown) instead of or in addition to displaying the support information on the display 22.

An input interface 24 is configured with, for example, a button, a trackball, a touch panel, and the like. The input interface 24 is used for a user such as an examiner to input a command to the ultrasound diagnostic apparatus 10.

The controller 26 includes at least one of a general-purpose processor (for example, a central processing unit (CPU) or the like) or a dedicated processor (for example, a graphics processing unit (GPU), an application specific integrated circuit (ASIC), a field-programmable gate array (FPGA), a programmable logic device, or the like). The controller 26 may be configured with a plurality of processing devices that are present at physically separated positions and that cooperate with each other, rather than being configured with one processing device. The controller 26 controls each unit of the ultrasound diagnostic apparatus 10 in accordance with an ultrasound diagnosis program stored in a memory 28, which will be described below.

The memory 28 includes a hard disk drive (HDD), a solid state drive (SSD), an embedded multi media card (eMMC), a read only memory (ROM), or the like. The memory 28 stores the ultrasound diagnosis program for operating each unit of the ultrasound diagnostic apparatus 10. The ultrasound diagnosis program can also be stored in, for example, a computer-readable non-transitory storage medium such as a universal serial bus (USB) memory or a CD-ROM. The ultrasound diagnostic apparatus 10 can read the ultrasound diagnosis program from such a storage medium and execute the ultrasound diagnosis program.

In addition, as shown in Fig. 1, the memory 28 stores a learning model 30 and a support information database (DB) 32.

The learning model 30 is, for example, a convolutional neural network (CNN) or the like, but may be any model as long as it exhibits functions to be described below. The learning model 30 is trained by using a combination of the ultrasound tomographic image and a cross-section classification (ground truth cross-section classification) of the ultrasound tomographic image as training data, to predict and output the cross-section classification of the input ultrasound tomographic image. In the present embodiment, training processing of the learning model 30 is performed by a training processing device different from the ultrasound diagnostic apparatus 10, and a sufficiently trained learning model 30 is stored in the memory 28. However, the ultrasound diagnostic apparatus 10 may include a training processing unit (not shown in Fig. 1), and the training processing unit may perform the training processing of the learning model 30. In this case, the ultrasound diagnostic apparatus 10 serves as a training processing device.

The details of the training processing of the learning model 30 are as follows. A processor of the training processing device inputs the ultrasound tomographic image as training data to the learning model 30. The learning model 30 predicts and outputs the cross-section classification of the input ultrasound tomographic image. The processor of the training processing device changes parameters in the learning model 30 such that a difference between output data of the learning model 30 and the ground truth cross-section classification included in the training data is reduced. By repeatedly performing such processing, the learning model 30 is trained. The learning model 30 that has been sufficiently trained can predict the cross-section classification of the input ultrasound tomographic image with high accuracy.

The learning model 30 according to the present embodiment has an activation function, such as a softmax function, in an output layer thereof and outputs, as the output data, a plurality of combinations of the cross-section classifications and the parameters indicating a probability that the input ultrasound tomographic image is the cross-section classification.

The support information DB 32 is a database that stores support information related to the operation of the ultrasound diagnostic apparatus 10 and used to support the formation of the targeted cross-section which is the cross-section classification of the ultrasound tomographic image that needs to be formed by the examiner. The support information is provided to the examiner.

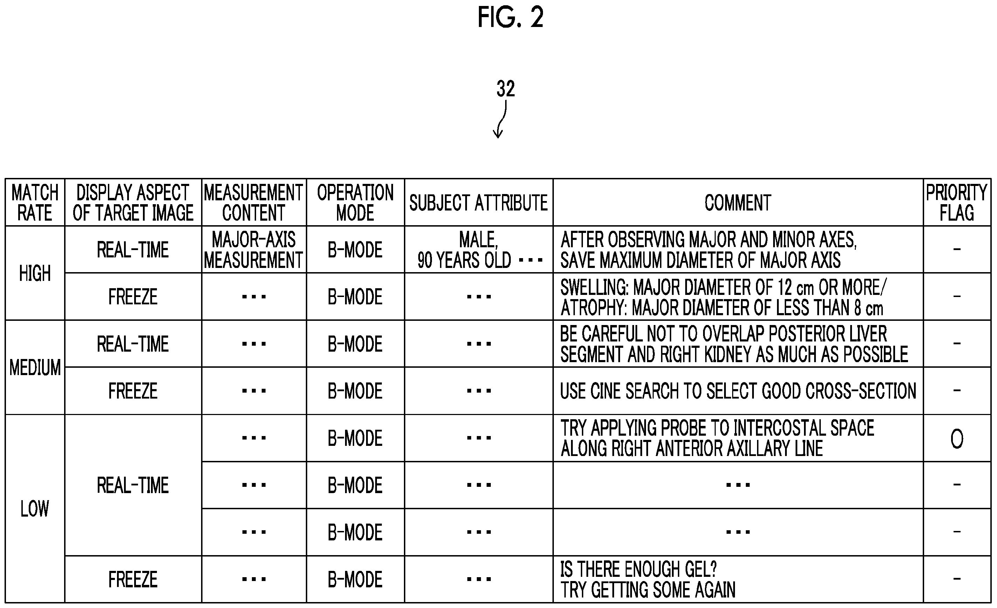

Fig. 2 is a conceptual diagram showing an example of the content of the support information DB 32. In the support information DB 32, different pieces of support information are associated with a plurality of different match rates from each other. In the example in Fig. 2, a comment as the support information is associated with the match rate. The match rate is a match rate between the cross-section classification of the ultrasound tomographic image (referred to as a target image in the present specification) formed by transmitting ultrasound waves to the subject in response to an instruction from the examiner and the targeted cross-section. For example, in a case where the cross-section classification of the target image and the targeted cross-section are the same, the match rate is considerably high, and in a case where the cross-section classification of the target image and the targeted cross-section are cross-sections completely different from each other, the match rate is considerably low. In a case where the cross-section classification of the target image and the targeted cross-section are different from each other but are close to each other (for example, in a case where the same site is shown but the cross-sections are slightly different or the like), the match rate is lower than that in a case where the cross-section classification of the target image and the targeted cross-section are the same, and the match rate is higher than that in a case where the cross-section classification of the target image and the targeted cross-section are cross-sections completely different from each other. The match rate may be said to indicate a probability that the cross-section classification of the target image is the targeted cross-section.

In the present embodiment, in the support information DB 32, the numerical range in which the match rate can be taken is divided into a plurality of match rate levels by one or a plurality of division threshold values. Then, in the support information DB 32, the support information is associated with a combination of the examiner level and the match rate level. In the example in Fig. 2, the numerical range in which the match rate can be taken is divided into a match rate level of "high" (for example, the match rate is 70% or more), a match rate level of "medium" (for example, the match rate is 50% or more and less than 70%), and a match rate level of "low" (for example, the match rate is less than 50%). In this case, the match rates of "70%" and "50%" are the division threshold values. Then, a different comment is associated with each match rate level as each piece of support information.

In the support information DB 32, the support information may be associated with each combination of the match rate and the display aspect of the target image. In the present embodiment, the display aspect of the target image is real-time or freeze. In the example in Fig. 2, a comment is associated with each combination of each match rate and the display aspect "real-time" of the target image, and a comment is associated with each combination of each match rate and the display aspect "freeze" of the target image.

In addition, in the support information DB 32, the support information may be associated with each combination of the match rate and a measurement content using the target image. The measurement content is a content of various measurements using the ultrasound image and is, for example, a major-axis measurement of the heart or the like. The measurement content may be designated by the examiner. In addition, in the ultrasound diagnostic apparatus 10, an examination protocol in which a series of examination processes and an examination execution condition for each examination process are set may be stored, and in this case, the measurement content may be determined based on the examination protocol selected by the examiner and the progress of the examination protocol.

Further, in the support information DB 32, the support information may be associated with each combination of the match rate and the operation mode of the ultrasound diagnostic apparatus 10. As described above, the operation modes of the ultrasound diagnostic apparatus 10 include the B-mode, the PW Doppler mode, the CW Doppler mode, the color Doppler mode, and the like, but the operation modes are not limited to these.

Furthermore, in the support information DB 32, the support information may be associated with each combination of the match rate and an attribute of the subject. Examples of the attribute of the subject include the sex, the weight, the disease condition, the country or region, and the facility (hospital or university) where the subject is located, but the attribute of the subject is not limited to these.

In the present embodiment, as shown in Fig. 2, a comment, which is the support information, is associated with a combination of the match rate, the display aspect of the target image, the measurement content using the target image, the operation mode of the ultrasound diagnostic apparatus 10, and the attribute of the subject. The aspect of the association between the match rate, the display aspect of the target image, the measurement content using the target image, the operation mode of the ultrasound diagnostic apparatus 10, the attribute of the subject, and the support information may be any aspect.

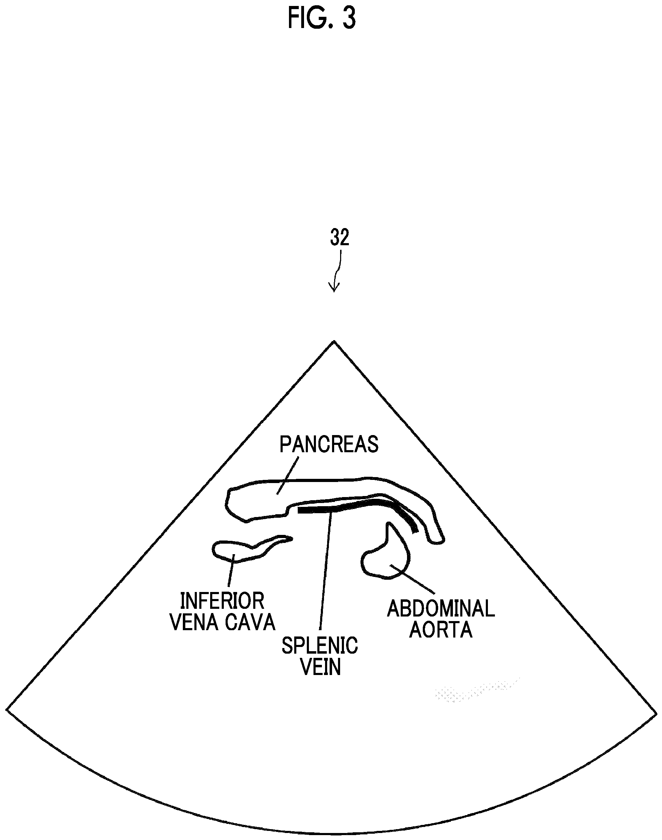

Fig. 3 is a diagram showing a first example of a reference image as the support information. In the example in Fig. 2, the comment is shown as the support information, but the support information stored in the support information DB 32 may be the reference image as shown in Fig. 3. The reference image may be, for example, an ultrasound tomographic image showing a targeted cross-section, a schematic diagram, an illustration, a schema diagram, a CT image, an MRI image, an image (including an illustration) showing an appropriate position of the ultrasound probe 12, or the like. As shown in Fig. 3, in the reference image, each site (pancreas, inferior vena cava, splenic vein, abdominal aorta, and the like) in the targeted cross-section may be clearly indicated by text, figures, and the like.

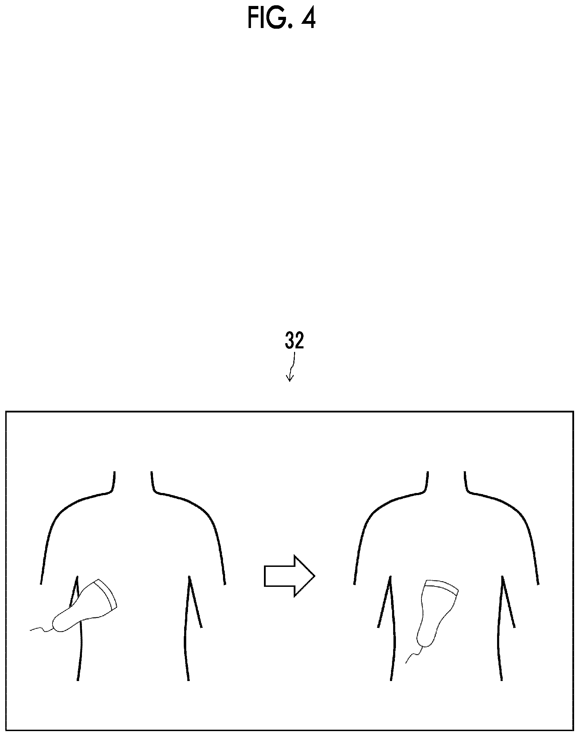

Fig. 4 is a diagram showing a second example of the reference image as the support information. In the support information DB 32, the reference image as shown in Fig. 4 as the support information may be associated with a combination of the match rate, a first cross-section classification, and a second cross-section classification other than the first cross-section classification. The reference image in Fig. 4 is a diagram in which the left side of an arrow indicates the position of the ultrasound probe 12 in a case where the ultrasound image of the first cross-section classification is formed, and the right side of the arrow indicates the position of the ultrasound probe 12 in a case where the ultrasound image of the second cross-section classification is formed. Therefore, in a case where the examiner wants to form the ultrasound image of the second cross-section classification as the targeted cross-section but mistakenly forms the ultrasound image of the first cross-section classification (that is, in a case where the ultrasound probe 12 is mistakenly applied as shown in the left side of Fig. 4), displaying such a reference image allows the examiner to easily understand how to change the position of the ultrasound probe 12 from the current position to an appropriate position. The reference image as shown in Fig. 4 may be a moving image that changes from the left side diagram to the right side diagram.

A method of using the support information DB 32 will be described below. In addition, a priority flag associated with each piece of support information shown in Fig. 2 will be described below.

Returning to Fig. 1, a support information editing unit 34 edits the support information stored in the support information DB 32 in response to an instruction from the user, such as the examiner of the ultrasound diagnostic apparatus 10 or an instructor who instructs the examiner, which is input via the input interface 24. The support information editing unit 34 changes, for example, the comment as the support information stored in the support information DB 32 in response to an instruction from the user. Alternatively, the support information editing unit 34 changes, for example, the reference image as the support information stored in the support information DB 32 in response to an instruction from the user.

A targeted cross-section specifying unit 36 specifies a cross-section type of the ultrasound tomographic image that needs to be formed by the examiner, that is, the targeted cross-section. In the present embodiment, the ultrasound diagnostic apparatus 10 stores the above-described examination protocol, and the targeted cross-section specifying unit 36 specifies the targeted cross-section based on the examination protocol selected by the examiner and the progress of the examination protocol. For example, in the examination protocol, it is assumed that a first examination process is an examination performed using an ultrasound image of a cross-section classification A, and a second examination process is an examination performed using an ultrasound image of a cross-section classification B. In this case, the targeted cross-section is the cross-section classification A in the first examination process, and the targeted cross-section is the cross-section classification B in the second examination process. In addition, the targeted cross-section specifying unit 36 may specify the targeted cross-section based on the examiner's instruction input via the input interface 24. That is, the targeted cross-section may be designated by the examiner.

A match rate calculation unit 38 calculates a match rate between the target image and the targeted cross-section specified by the targeted cross-section specifying unit 36 by executing the cross-section recognition processing on the target image. Specifically, the match rate calculation unit 38 inputs the target image to the trained learning model 30 that executes the cross-section recognition processing. Consequently, the learning model 30 predicts and outputs the cross-section classification of the target image. As described above, the learning model 30 outputs the cross-section classification and the parameter indicating the probability that the target image is the cross-section classification. For example, the cross-section classification A is 80%, the cross-section classification B is 15%, and a cross-section classification C is 5%. In the present embodiment, as the match rate between the target image and the targeted cross-section, the parameter indicating the probability of each cross-section classification output by the learning model 30 is employed. For example, in a case where the output data of the learning model 30 is the above-described example, the match rate between the target image and the targeted cross-section is 80% in a case where the targeted cross-section is the cross-section classification A, the match rate between the target image and the targeted cross-section is 15% in a case where the targeted cross-section is the cross-section classification B, and the match rate between the target image and the targeted cross-section is 5% in a case where the targeted cross-section is the cross-section classification C.

Hereinafter, details of output processing of the support information by the output unit 20 will be described. In addition, in the following description, it is assumed that the content of the support information DB 32 is the content shown in Fig. 2.

The output unit 20 outputs, based on the support information DB 32, the support information with the content tailored to the match rate between the cross-section classification of the target image and the targeted cross-section, which is calculated by the match rate calculation unit 38. For example, in a case where the match rate calculated by the match rate calculation unit 38 is 80% (that is, the match rate level is "high"), the output unit 20 outputs the support information associated with the match rate level "high" in the support information DB 32 (for example, the comments "After observing major and minor axes, save the maximum diameter of the major axis", "Swelling: a major diameter of 12 cm or more/atrophy: a major diameter of less than 8 cm", or the reference image). In addition, in a case where the match rate calculated by the match rate calculation unit 38 is 60% (that is, the match rate level is "medium"), the output unit 20 outputs the support information associated with the match rate level "medium" in the support information DB 32 (for example, the comments "Be careful not to overlap the posterior liver segment and the right kidney as much as possible", "Use cine search to select a good cross-section", or the reference image). Further, in a case where the match rate calculated by the match rate calculation unit 38 is 20% (that is, the match rate level is "low"), the output unit 20 outputs the support information associated with the match rate level "low" in the support information DB 32 (for example, the comments "Try applying the probe to the intercostal space along the right anterior axillary line", "Is there enough gel? Try getting some again", or the reference image).

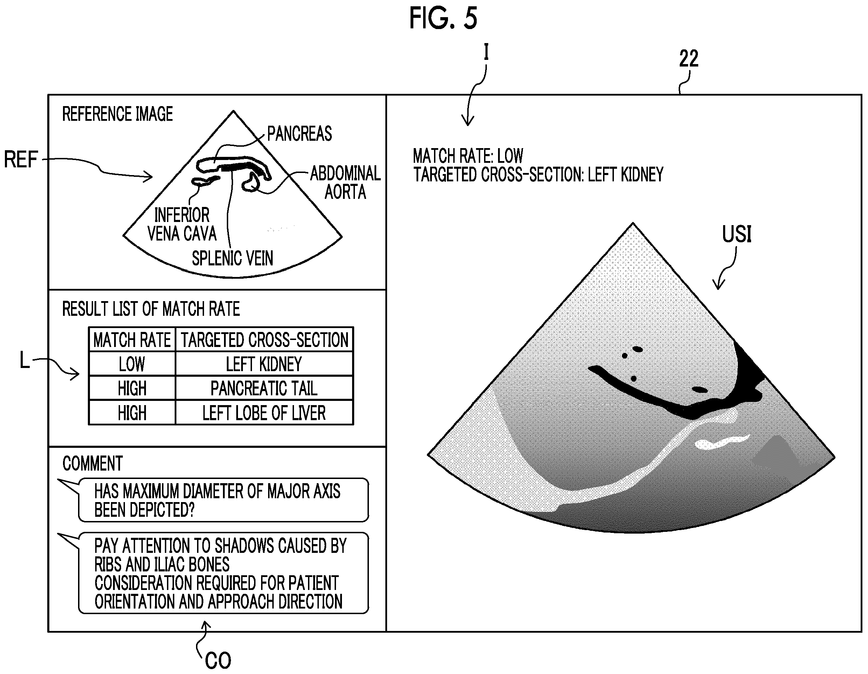

Fig. 5 is a diagram showing an example of a support information display screen displayed on the display 22 by the output unit 20. In the example in Fig. 5, a comment CO as the support information and a reference image REF as the support information are displayed on a support information output screen together with a target image USI. Additionally, on the support information output screen, reference information I such as the match rate between the cross-section classification of the target image and the targeted cross-section, which is calculated by the match rate calculation unit 38, and the targeted cross-section specified by the targeted cross-section specifying unit 36 may be displayed.

As described above, according to the present embodiment, since the support information tailored to the match rate between the cross-section classification of the target image and the targeted cross-section is provided to the examiner, it is possible to provide the support information particularly suitable for the examiner.

In a case where the support information is associated with each combination of the match rate and the display aspect of the target image in the support information DB 32, the output unit 20 may output the support information with the content tailored to the match rate between the cross-section classification of the target image and the targeted cross-section and to the display aspect (that is, real-time or freeze) of the target image designated by the examiner. For example, in a case where the match rate calculated by the match rate calculation unit 38 is 80% (that is, the match rate level is "high") and the display aspect of the target image is "real-time", the output unit 20 outputs the support information associated with the match rate level "high" and the display aspect "real-time" of the target image in the support information DB 32 (for example, the comment "After observing major and minor axes, save the maximum diameter of the major axis" or the reference image). Meanwhile, in a case where the match rate calculated by the match rate calculation unit 38 is 80% (that is, the match rate level is "high") and the display aspect of the target image is "freeze", the output unit 20 outputs the support information associated with the match rate level "high" and the display aspect "freeze" of the target image in the support information DB 32 (for example, the comment "Swelling: a major diameter of 12 cm or more/atrophy: a major diameter of less than 8 cm" or the reference image).

In a case where the support information is associated with each combination of the match rate and the measurement content using the target image in the support information DB 32, the output unit 20 may output the support information with the content tailored to the match rate between the cross-section classification of the target image and the targeted cross-section and to the measurement content specified by the examiner designation or the examination protocol. For example, in a case where the match rate calculated by the match rate calculation unit 38 is 80% (that is, the match rate level is "high") and the measurement content using the target image is "major-axis measurement", the output unit 20 outputs the support information associated with the match rate level "high" and the measurement content "major-axis measurement" in the support information DB 32 (for example, the comment "After observing major and minor axes, save the maximum diameter of the major axis" or the reference image).

In a case where the support information is associated with each combination of the match rate and the operation mode of the ultrasound diagnostic apparatus 10 in the support information DB 32, the output unit 20 may output the support information with the content tailored to the match rate between the cross-section classification of the target image and the targeted cross-section and to the current operation mode of the ultrasound diagnostic apparatus 10. For example, in a case where the match rate calculated by the match rate calculation unit 38 is 80% (that is, the match rate level is "high") and the current operation mode of the ultrasound diagnostic apparatus 10 is the B-mode, the output unit 20 outputs the support information associated with the match rate level "high" and the operation mode "B-mode" in the support information DB 32 (for example, the comments "After observing major and minor axes, save the maximum diameter of the major axis", "Swelling: a major diameter of 12 cm or more/atrophy: a major diameter of less than 8 cm", or the reference image).

In a case where the support information is associated with each combination of the match rate and the attribute of the subject in the support information DB 32, the output unit 20 may output the support information with the content tailored to the match rate between the cross-section classification of the target image and the targeted cross-section and to the attribute of the subject. The attribute of the subject corresponding to the target image may be input to the ultrasound diagnostic apparatus 10 by the examiner or may be recorded in the above-described examination protocol. For example, in a case where the match rate calculated by the match rate calculation unit 38 is 80% (that is, the match rate level is "high") and the attribute of the subject is a 90-year-old male, the output unit 20 outputs the support information associated with the match rate level "high" and the subject attribute "male, 90 years old" in the support information DB 32 (for example, the comment "After observing major and minor axes, save the maximum diameter of the major axis" or the reference image).

In addition, in a case where the support information (refer to Fig. 4) is associated with the combination of the match rate, the first cross-section classification, and the second cross-section classification in the support information DB 32, the output unit 20 may output the support information with the content tailored to the match rate between the cross-section classification of the target image and the targeted cross-section, to the cross-section type of the target image as the first cross-section classification, and to the targeted cross-section as the second cross-section classification. In detail, the output unit 20 specifies the support information associated with the combination of the match rate calculated by the match rate calculation unit 38, the cross-section type of the target image as the first cross-section classification, and the targeted cross-section specified by the targeted cross-section specifying unit 36 as the second cross-section classification, from the support information DB 32, and outputs the specified support information. The cross-section type of the target image here means a cross-section classification with the highest probability of relevance among probabilities of relevance for a plurality of cross-section classifications calculated by the match rate calculation unit 38.

Specifically, it is assumed that the reference image as shown in Fig. 4 is associated as the support information with the combination of the match rate level "low", the first cross-section classification "cross-section classification A", and the second cross-section classification "cross-section classification B". That is, in the reference image of Fig. 4, it is assumed that the left side of the arrow is a diagram indicating the position of the ultrasound probe 12 in a case where the ultrasound image of the first cross-section classification "cross-section classification A" is formed, and the right side of the arrow is a diagram indicating the position of the ultrasound probe 12 in a case where the ultrasound image of the second cross-section classification "cross-section classification B" is formed. In this case, in a case where the match rate calculated by the match rate calculation unit 38 is 20% (that is, the match rate level is "low"), and the cross-section classification of the target image is the cross-section classification A and the targeted cross-section is the cross-section classification B, the output unit 20 outputs the reference image shown in Fig. 4 as the support information.

In a case where the output unit 20 outputs the support information, the examiner changes the position of the ultrasound probe 12 or changes the settings of the ultrasound diagnostic apparatus 10 based on the output support information. Consequently, the match rate between the cross-section classification of the target image and the targeted cross-section may be improved. In this case, it can be considered that the output support information is effective. On the other hand, even in a case where the support information is output, the match rate between the cross-section classification of the target image and the targeted cross-section may not be improved. In this case, it can be considered that the output support information is not very effective.

Therefore, the match rate calculation unit 38 may calculate, for the same targeted cross-section, the match rate with the cross-section classification of the target image again after the support information has been output by the output unit 20. In a case where the match rate after the support information has been output is higher than the match rate before the support information is output, the priority flag (refer to Fig. 2) may be assigned to the support information in the support information DB 32. On the other hand, in a case where the match rate after the support information has been output is the same as or lower than the match rate before the support information is output, the match rate calculation unit 38 does not assign the priority flag to the support information. As a result, the support information with the priority flag assigned is considered effective information, that is, information that can improve the match rate between the cross-section classification of the target image and the targeted cross-section.

Then, the output unit 20 may preferentially output the support information with the priority flag assigned, in a case where a plurality of pieces of support information are associated with the match rate in the support information DB 32, over the support information with no priority flag assigned. For example, as shown in Fig. 2, it is assumed that, among a plurality of pieces of support information associated with the match rate level "low", the priority flag is assigned to the comment "Try applying the probe to the intercostal space along the right anterior axillary line" and the priority flag is not assigned to the comment "Is there enough gel? Try getting some again". Here, in a case where the match rate calculated by the match rate calculation unit 38 is 20% (that is, the match rate level is "low"), the output unit 20 preferentially outputs the comment "Try applying the probe to the intercostal space along the right anterior axillary line", which is the support information with the priority flag assigned, among the pieces of support information associated with the match rate level "low" in the support information DB 32. As a result, it is possible to preferentially output the support information considered to be more effective.

In the ultrasound diagnostic apparatus 10, a case is considered in which an examination is performed along a plurality of examination processes, each forming an ultrasound tomographic image of a different targeted cross-section. For example, the examination follows the above-described examination protocol. In this case, the examiner proceeds with the examination while forming the ultrasound tomographic image of the targeted cross-section defined in each examination process, for the examination process. The match rate calculation unit 38 calculates, each time the examiner forms the ultrasound tomographic image (target image) in each examination process, the match rate between the cross-section type of the target image formed in the examination process and the targeted cross-section related to the examination process. That is, the match rate is calculated for each examination process. Here, the match rate calculation unit 38 stores the match rate calculated in each examination process in the memory 28 in association with each examination process.

The output unit 20 may output the calculated match rate for a completed examination process during the examination along the plurality of examination processes. For example, as shown in Fig. 5, the output unit 20 may read the calculated match rate for the completed examination process from the memory 28 and display a list L of the match rate for each examination process on the display 22. In the example of Fig. 5, in the plurality of examination processes, an ultrasound tomographic image of a targeted cross-section "left lobe of the liver" is formed in an examination process 1, an ultrasound tomographic image of a targeted cross-section "pancreatic tail" is formed in an examination process 2, and an ultrasound tomographic image of a targeted cross-section "left kidney" is formed in an examination process 3. According to the list L, the examiner can confirm that the match rate between the cross-section classification of the target image and the targeted cross-section in the examination processes 1 and 2 is "high" during the execution of the examination process 3.

In addition, a setting value (proper setting value) of the ultrasound diagnostic apparatus 10 that is appropriate for forming the ultrasound image of the targeted cross-section may be prepared in advance for each examination process, and the output unit 20 may output the comment as the support information in a case where the setting value set by the examiner and the proper setting value deviate from each other by a predetermined amount or more. For example, a comment such as "The gain is 40. Isn't that too low?" is output.

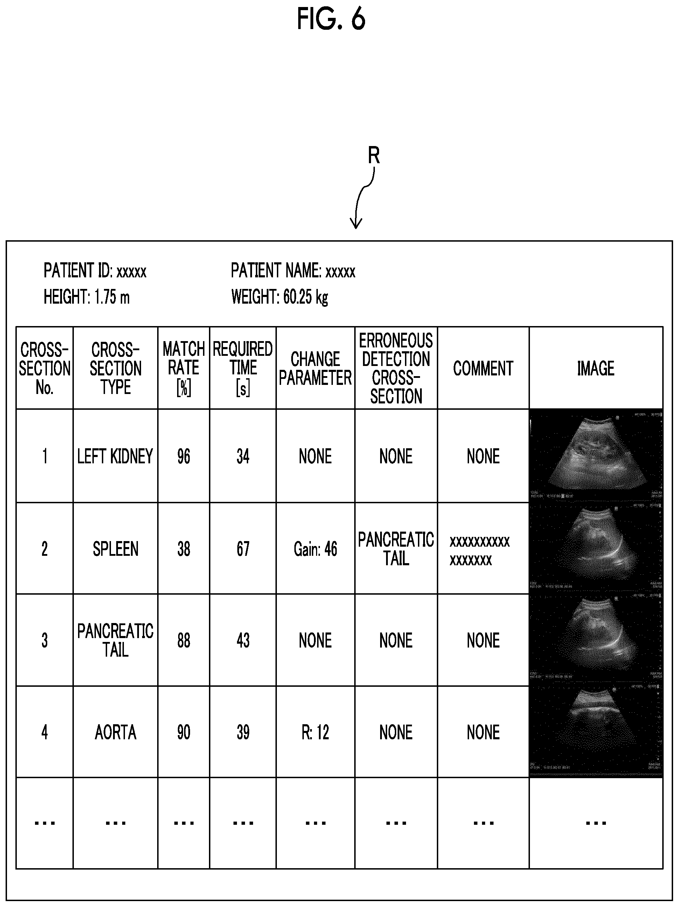

The output unit 20 may output an examination report after the plurality of examination processes have ended. Fig. 6 is a diagram showing an example of an examination report R. The examination report R may be output in a form of being displayed on the display 22 or may be output by being printed on a print medium such as paper. The examination report R allows, for example, an instructor who instructs a beginner or the like to retrospectively review the content of the examination across the plurality of examination processes performed by the beginner.

The examination report R includes a plurality of item values for a plurality of items for each of the plurality of examination processes. In particular, the plurality of items of the examination report R include the match rate between the cross-section classification of the target image and the targeted cross-section and the support information output in a case of attempting to form an ultrasound tomographic image of a targeted cross-section related to the examination process. The examination report R shown in Fig. 6 has "cross-section No.", "cross-section type", "match rate", "required time", "change parameter", "erroneous detection cross-section", "comment", and "image" as the plurality of items.

The item "cross-section No." is a number for identifying the examination process.

The item "cross-section type" indicates the targeted cross-section in the examination process.

The item "match rate" is a match rate calculated by the match rate calculation unit 38 in the examination process.

The item "required time" is a time required by the examiner for the examination in the examination process.

For the time required for the examination in each examination process, for example, the time from the start of the examination process to the end of the examination process is automatically measured by a timing function of the ultrasound diagnostic apparatus 10.

The item "change parameter" is a setting value (particularly, a setting value related to the formation of the ultrasound image or the image quality correction) of the ultrasound diagnostic apparatus 10 changed by the examiner in the examination process. Examples of such a setting value include a gain, a depth, a frame rate, a velocity range, an ROI size, a baseline, a sample volume, an angle, and a reliability degree of SWE/SWM/ATT.

The item "erroneous detection cross-section" is a cross-section classification in a case where the cross-section classification determined by the match rate calculation unit 38 to have the highest match rate with the cross-section classification of the target image in the examination process is a cross-section classification other than the targeted cross-section of the examination process.

The item "comment" is a comment output as the support information in the examination process.

The item "image" is a target image formed by the examiner in the examination process.

In addition to the above-described information, the examination report R may include information (such as an examiner ID or a name) for identifying the attribute of the subject or an examiner who has performed the examination in the plurality of examination processes. These pieces of information are input to the ultrasound diagnostic apparatus 10 by the examiner prior to the examination in the plurality of examination processes.

In the examination report R, the item values need not be associated with all the items for each examination process. In particular, the output unit 20 determines the item to be associated with the item value for the examination process, according to the match rate for the examination process. In other words, the output unit 20 outputs the examination report R that associates the information on the item tailored to the match rate corresponding to each targeted cross-section with the targeted cross-section. For example, the output unit 20 need not include, in the examination report R, the item value for at least one of the item "change parameter" or the item "erroneous detection cross-section" for the examination process in which the match rate level is "high" (for example, the match rate is 70% or more) (in this case, "-" or the like may be output to the corresponding field, or nothing may be displayed). This is because, in a case where the match rate is high, these items are not very important. On the other hand, the output unit 20 may include, in the examination report R, the item value for at least one of the item "change parameter" or the item "erroneous detection cross-section" for the examination process in which the match rate level is "medium" (for example, the match rate is 50% or more and less than 70%) or the match rate level is "low" (for example, the match rate is less than 50%).

The outline of the ultrasound diagnostic apparatus according to the present embodiment is as described above. The ultrasound diagnostic apparatus according to the present embodiment is not limited to the above-described embodiment, and various changes can be made without departing from the gist thereof.

The transmission and reception unit 14, the signal processing unit 16, the image forming unit 18, the output unit 20, the support information editing unit 34, the targeted cross-section specifying unit 36, and the match rate calculation unit 38, which are shown in Fig. 1, are functional blocks representing functions implemented by a processor. The processor includes at least one of a general-purpose processing device (for example, a CPU or the like) or a dedicated processing device (for example, a GPU, an ASIC, an FPGA, a programmable logic device, or the like). The processor may be configured with a plurality of processing devices that are present at physically separated positions and that cooperate with each other, rather than being configured with one processing device. In addition, each of the above-described units may be implemented by cooperation between hardware, such as a processor, and software.

### Explanation of References

10: ultrasound diagnostic apparatus
12: ultrasound probe
14: transmission and reception unit
16: signal processing unit
18: image forming unit
20: output unit
22: display
24: input interface
26: controller
28: memory
30: learning model
32: support information DB
34: support information editing unit
36: targeted cross-section specifying unit
38: match rate calculation unit

## Claims

1. An ultrasound diagnostic apparatus (10) comprising:
a targeted cross-section specifying unit (36) that specifies a targeted cross-section which is a cross-section classification of an ultrasound tomographic image that needs to be formed;
a match rate calculation unit (38) that executes cross-section recognition processing on a target image, which is an ultrasound tomographic image formed in response to an instruction from an examiner, to calculate a match rate between a cross-section classification of the target image and the targeted cross-section; and
an output unit (20) that outputs, based on a support information database in which support information related to an operation of the ultrasound diagnostic apparatus (10) and used to support formation of a targeted cross-section, which is a cross-section classification of an ultrasound tomographic image that needs to be formed by the examiner, is associated with each match rate, support information with a content tailored to the match rate calculated by the match rate calculation unit (38).

2. The ultrasound diagnostic apparatus (10) according to claim 1,
wherein, in the support information database, the support information is associated with each combination of the match rate and a display aspect of the target image, and
the output unit (20) outputs support information with a content tailored to the match rate calculated by the match rate calculation unit (38) and to the display aspect of the target image.

3. The ultrasound diagnostic apparatus (10) according to claim 1,
wherein, in the support information database, the support information is associated with a combination of the match rate and a measurement content using the target image, and
the output unit (20) outputs support information with a content tailored to the match rate calculated by the match rate calculation unit (38) and to the measurement content.

4. The ultrasound diagnostic apparatus (10) according to claim 1,
wherein, in the support information database, the support information is associated with a combination of the match rate and an operation mode of the ultrasound diagnostic apparatus (10), and
the output unit (20) outputs support information with a content tailored to the match rate calculated by the match rate calculation unit (38) and to the operation mode of the ultrasound diagnostic apparatus (10).

5. The ultrasound diagnostic apparatus (10) according to claim 1,
wherein, in the support information database, the support information is associated with a combination of the match rate and an attribute of a subject, and
the output unit (20) outputs support information with a content tailored to the match rate calculated by the match rate calculation unit (38) and to the attribute of the subject.

6. The ultrasound diagnostic apparatus (10) according to claim 1,
wherein, in the support information database, the support information is associated with a combination of the match rate, a first cross-section classification, and a second cross-section classification other than the first cross-section classification, and
the output unit (20) outputs support information with a content tailored to the match rate calculated by the match rate calculation unit (38), to the cross-section classification of the target image as the first cross-section classification, and to the targeted cross-section as the second cross-section classification.

7. The ultrasound diagnostic apparatus (10) according to any one of claims 1 to 6, further comprising:
a support information editing unit (34) that edits the support information in response to an instruction from a user of the ultrasound diagnostic apparatus (10).

8. The ultrasound diagnostic apparatus (10) according to claim 1,
wherein the match rate calculation unit (38) calculates the match rate for the same targeted cross-section again after the support information has been output, and assigns, in a case where the match rate after the support information has been output is higher than the match rate before the support information is output, a priority flag to the support information in the support information database, and
the output unit (20) preferentially outputs the support information with the priority flag assigned, in a case where a plurality of pieces of the support information are associated with the match rate in the support information database, over support information with no priority flag assigned.

9. The ultrasound diagnostic apparatus (10) according to claim 1,
wherein, during an examination along a plurality of examination processes, each forming an ultrasound tomographic image of a different targeted cross-section, the match rate calculation unit (38) calculates the match rate for each examination process and stores the match rate in a memory, and
the output unit (20) outputs the calculated match rate for a completed examination process during the examination along the plurality of examination processes.

10. The ultrasound diagnostic apparatus (10) according to claim 1,
wherein the output unit (20) outputs, after a plurality of examination processes, each forming an ultrasound tomographic image of a different targeted cross-section, have ended, an examination report that includes, for each of the plurality of examination processes, item values for a plurality of items including the match rate and the support information output in a case of attempting to form an ultrasound tomographic image of a targeted cross-section related to the examination process, the examination report associating information on the item tailored to the match rate corresponding to each targeted cross-section with the targeted cross-section.
